# FASCICULE DE BREVET EUROPEEN

(11) **EP 1 564 204 B1**
(45) Date de publication et mention de la délivrance du brevet: **02.01.2008**
(21) Numéro de dépôt: 05290309.3
(22) Date de dépôt: 11.02.2005
(51) Int. Cl.: C07C 253/30

(54) **Nouveau procédé de synthese du (7-methoxy-3,4-dihydro-1-naphtalenyl) acetonitrile et application à la synthese de l' aglomelatine**
Neues Verfahren zur Herstellung von (7-Methoxy-3,4-dihydro-1-naphtalenyl) acetonitril und dessen Anwendung in der Synthese von Agomelatin
New process for synthesizing (7-methoxy-3,4-dihydro-1-naphtalenyl) acetonitrile and its application in the synthesis of agomelatine

(30) Priorité: 13.02.2004 FR 0401436
(43) Date de publication de la demande: 17.08.2005
(73) Titulaire: Les Laboratoires Servier, 92415 Courbevoie Cédex (FR)
(72) Inventeur: Souvie, Jean-Claude, 76600 Le Havre (FR); Gonzalez Blanco, Isaac, 45002 Toledo (ES)

(56) Documents cités:
- EP-A- 0 447 285
- US-A- 3 931 188
- US-A- 3 992 403

## Description

La présente invention concerne un procédé de synthèse industriel du (7-méthoxy-3,4-dihydro-1-naphtalényl)acétonitrile, et son application à la production industrielle de l'agomélatine ou *N*-[2-(7-méthoxy-1-naphtyl)éthyl]acétamide.

Plus spécifiquement, la présente invention concerne un procédé de synthèse industriel du composé de formule (I) :

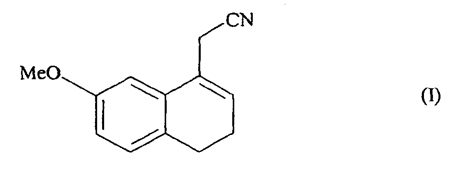

Le composé de formule (I) obtenu selon le procédé de l'invention est utile dans la synthèse de l'agomélatine ou *N*-[2-(7-méthoxy-1-naphtyl)éthyl]acétamide de formule (II) :

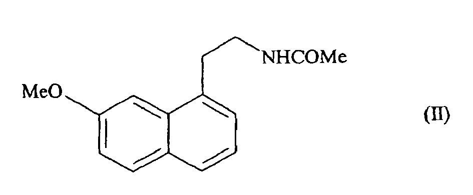

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte-tenu de l'intérêt pharmaceutique de ce composé, il était important de pouvoir y accéder avec un procédé de synthèse industrielle performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone avec un rendement moyen inférieur à 30%.

Ce procédé implique l'action du bromoacétate d'éthyle, suivie d'une aromatisation et saponification pour conduire à l'acide correspondant, qui est ensuite transformé en acétamide puis déshydraté pour conduire au (7-méthoxy-1-naphtyl)acétonitrile, suivie d'une réduction puis de la condensation du chlorure d'acétyle.

Transposé à l'échelle industrielle, il a rapidement été mis en évidence des difficultés de mise en oeuvre de ce procédé dues principalement à des problèmes de reproductibilité de la première étape constituée par l'action du bromoacétate d'éthyle sur la 7-méthoxy-1-tétralone selon la réaction de Réformatsky conduisant au (7-méthoxy-3,4-dihydro-1(2*H*)-naphtalenylidène)éthanoate d'éthyle.
De plus, l'étape suivante d'aromatisation du (7-méthoxy-3,4-dihydro-1(2*H*)-naphtalenylidène)éthanoate d'éthyle était souvent partielle et conduisait, après saponification à un mélange de produits difficilement purifiable.

La demanderesse a présentement mis au point un nouveau procédé de synthèse industrielle qui conduit, de façon reproductible et sans nécessiter de purification laborieuse, à l'agomélatine avec une pureté qui est compatible avec son utilisation comme principe actif pharmaceutique.

Une alternative aux difficultés rencontrées avec le procédé décrit dans le brevet EP 0 447 285 a été obtenue en condensant directement un dérivé cyano sur la 7-méthoxy-1-tétralone. Il fallait de plus que le composé de condensation obtenu puisse être facilement soumis à une aromatisation afin de conduire au (7-méthoxy-1-naphtyl)acétonitrile.

Il est apparu que le (7-méthoxy-3,4-dihydro-1-naphtalényl)acétonitrile constituerait un intermédiaire de synthèse idéal répondant aux exigences requises de synthèse directe à partir de la 7-méthoxy-1-tétralone et serait un excellent substrat pour l'étape d'aromatisation.

Des condensations directes de tétralones avec l'acétonitrile ou des dérivés d'acétonitrile sont décrites dans la littérature. En particulier, le brevet US 3,992,403 décrit la condensation de cyanométhylphosphonate sur la 6-fluoro-1-tétralone, et le brevet US 3,931,188 décrit la condensation de l'acétonitrile sur la tétralone conduisant à l'intermédiaire cyané qui est directement engagé dans la réaction suivante.
Appliqué à la 7-méthoxy-1-tétralone, la condensation de l'acétonitrile conduit à un mélange d'isomères « exo » majoritaire et « endo » minoritaire selon la figure 1 : 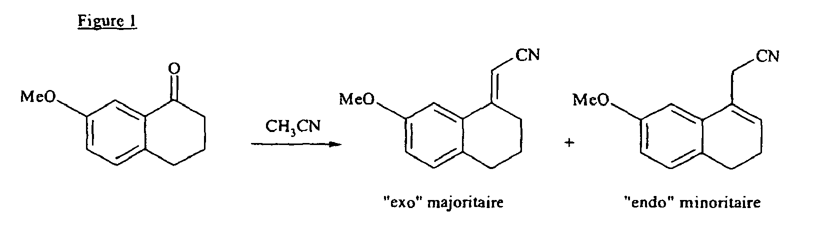 l'obtention d'un tel mélange nécessitant des conditions ultérieures d'aromatisation drastiques non compatibles avec les exigences industrielles pour poursuivre la synthèse de l'agomélatine.

La demanderesse a présentement mis au point un nouveau procédé de synthèse industrielle permettant d'obtenir le (7-méthoxy-3,4-dihydro-1-naphtalényl)acétonitrile de façon reproductible et sans nécessiter de purification laborieuse, en particulier exempt de l'impureté « exo » de formule (III) : 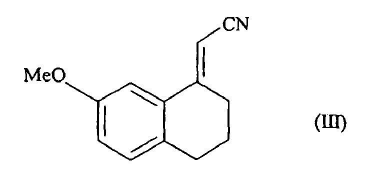 qui ne peut être soumis à une réaction d'aromatisation dans des conditions opératoires compatibles avec les exigences industrielles afin de poursuivre la synthèse de l'agomélatine.

Plus spécifiquement, la présente invention concerne un procédé de synthèse industriel du composé de formule (I) : 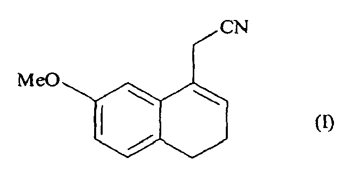 caractérisé en ce que l'on met en réaction la 7-méthoxy-1-tétralone de formule (IV) : 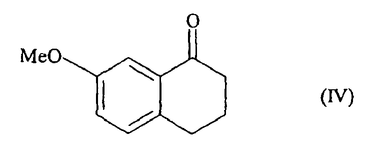 avec l'acide cyanoacétique de formule (V) : 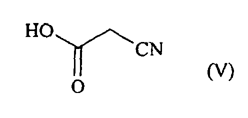 dans des conditions d'élimination de l'eau formée, en présence d'une quantité catalytique du composé de formule (VI) : 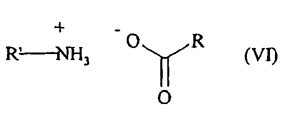 dans laquelle R et R', identiques ou différents, représentent chacun un groupement alkyle (C₃-C₁₀) linéaire ou ramifié, un groupement aryle non substitué ou substitué, ou un groupement arylalkyle (C₁-C₆) linéaire ou ramifié non substitué ou substitué,
pour conduire au composé de formule (I) après filtration et lavage par une solution basique, composé de formule (I) que l'on isole sous la forme d'un solide après recristallisation,
étant entendu que :
- par aryle on entend un groupement phényle, naphtyle ou biphényle,
- le terme « substitué » affecté aux expressions « aryle » et « arylalkyle » signifie que la partie aromatique de ces groupements peut être substituée par 1 à 3 groupements, identiques ou différents, choisis parmi alkyle (C₁-C₆) linéaire ou ramifié, hydroxy, et alkoxy (C₁-C₆) linéaire ou ramifié.

Plus particulièrement, l'eau formée est éliminée par distillation. On utilise préférentiellement un solvant de réaction ayant une température d'ébullition supérieure ou égale à celle de l'eau et encore plus préférentiellement formant un azéotrope avec l'eau comme par exemple le xylène, le toluène, l'anisole, l'éthylbenzène, le tétrachloroéthylène, le cyclohexène, ou le mésitylène.

De façon préférée, la réaction est réalisée au reflux du toluène ou du xylène, et plus préférentiellement au reflux du toluène.

Avantageusement, l'un des groupements R ou R' du catalyseur utilisé représente un groupement alkyle (C₃-C₁₀) linéaire ou ramifié, et l'autre représente un groupement aryle ou arylalkyle. Plus particulièrement, un catalyseur préféré est celui de formule (VIₐ) : 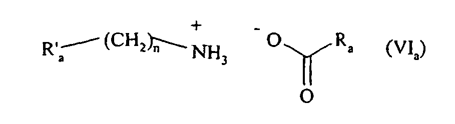 dans laquelle R'ₐ représente un groupement phényle non substitué ou substitué par un ou plusieurs groupements alkyle (C₁-C₆) linéaire ou ramifié, n vaut 0 ou 1, et Rₐ représente un groupement alkyle (C₃-C₁₀) linéaire.

Très avantageusement, R'ₐ représente un groupement phényle non substitué ou substitué et plus particulièrement un groupement phényle non substitué.

Le groupement Rₐ préféré est le groupement hexyle.

Avantageusement, n vaut 1.

Le catalyseur préféré utilisé dans le procédé selon l'invention est l'heptanoate de benzylammonium de formule (VII) :

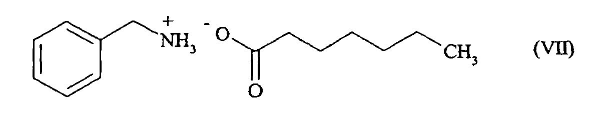

Avantageusement, le composé de formule (I) est obtenu après filtration et lavage par une solution basique organique ou minérale comme NaOH, KOH, Ca(OH)₂, Sr(OH)₂, ou NH₄OH, et plus préférentiellement par une solution d'hydroxyde de sodium.

Ce procédé est particulièrement intéressant pour les raisons suivantes :
- il permet d'obtenir à l'échelle industrielle le composé « endo » de façon exclusive. Ce résultat est tout à fait surprenant lorsqu'on considère la littérature concernant ce type de réaction qui fait le plus souvent état de l'obtention de mélanges « exo » / « endo » (Tetrahedron, 1966, 22, 3021-3026). Ce résultat provient de l'utilisation dans la réaction d'un catalyseur de formule (VI) en lieu et place des acétates d'ammonium couramment utilisés dans ces réactions (Bull. Soc. Chim. Fr., 1949, 884-890).
- le taux de transformation obtenu est très élevé, supérieur à 97% contrairement à ce qui a pu être observé avec l'utilisation d'acide acétique pour lequel ce taux ne dépasse pas 75%.

Le composé de formule (I) ainsi obtenu est nouveau et est utile en tant qu'intermédiaire de synthèse de l'agomélatine dans laquelle il est soumis à une réaction d'aromatisation suivie d'une réaction de réduction puis de couplage avec l'anhydride acétique.

Les exemples ci-dessous illustrent l'invention, mais ne la limitent en aucune façon.

### Exemple 1 : (7-Méthoxy-3,4-dihydro-1-naphtalényl)acétonitrile

Dans un réacteur de 670 1 sont introduits 85,0 kg de 7-méthoxy-1-tétralone, 60,3 kg d'acide cyanoacétique et 15,6 kg d'acide heptanoïque dans du toluène en présence de 12,7 kg de benzylamine. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 90% et une pureté chimique supérieure à 99%.
*Point de fusion : 48-50°C*

### Exemple 2 : (7-Méthoxy-3,4-dihydro-1-naphtalényl)acétonitrile

Dans un réacteur de 670 1 sont introduits 85,0 kg de 7-méthoxy-1-tétralone, 60,3 kg d'acide cyanoacétique et 15,6 kg d'acide heptanoïque dans du toluène en présence de 11,0 kg d'aniline. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 87% et une pureté chimique supérieure à 99%.
*Point de fusion : 48-50°C*

## Revendications

1. Procédé de synthèse industriel du composé de formule (I) 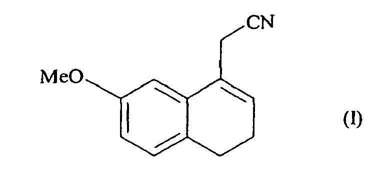 **caractérisé en ce que** l'on met en réaction la 7-méthoxy-1-tétralone de formule (IV) : 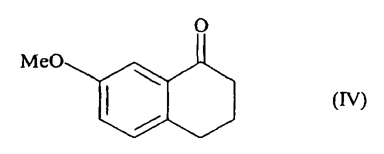 avec l'acide cyanoacétique de formule (V) : 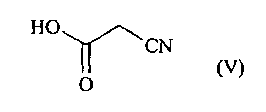 dans des conditions d'élimination de l'eau formée, en présence d'une quantité catalytique du composé de formule (VI) : 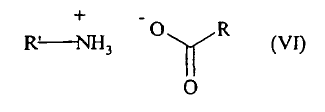 dans laquelle R et R', identiques ou différents, représentent chacun un groupement alkyle (C₃-C₁₀) linéaire ou ramifié, un groupement aryle non substitué ou substitué, ou un groupement arylalkyle (C₁-C₆) linéaire ou ramifié non substitué ou substitué,
pour conduire au composé de formule (I) après filtration et lavage par une solution basique, composé de formule (I) que l'on isole sous la forme d'un solide après recristallisation,
étant entendu que :
- par aryle on entend un groupement phényle, naphtyle ou biphényle,
- le terme « substitué » affecté aux expressions « aryle » et « arylalkyle » signifie que la partie aromatique de ces groupements peut être substituée par 1 à 3 groupements, identiques ou différents, choisis parmi alkyle (C₁-C₆) linéaire ou ramifié, hydroxy, et alkoxy (C₁-C₆) linéaire ou ramifié.

2. Procédé de synthèse du composé de formule (I) selon la revendication 1, **caractérisé en ce que** la réaction est réalisée au reflux du toluène.

3. Procédé de synthèse du composé de formule (I) selon la revendication 1, **caractérisé en ce que** le catalyseur utilisé est le composé de formule (VIₐ) : 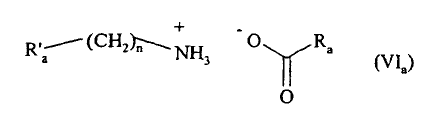 dans laquelle R'ₐ représente un groupement phényle non substitué ou substitué par un ou plusieurs groupements alkyle (C₁-C₆) linéaire ou ramifié, n vaut 0 ou 1, et Rₐ représente un groupement alkyle (C₃-C₁₀) linéaire.

4. Procédé de synthèse du composé de formule (I) selon la revendication 1, **caractérisé en ce que** R représente un groupement hexyle.

5. Procédé de synthèse du composé de formule (I) selon la revendication 1, **caractérisé en ce que** R' représente un groupement benzyle.

6. Procédé de synthèse du composé de formule (I) selon la revendication 1, **caractérisé en ce que** le catalyseur utilisé est l'heptanoate de benzylammonium de formule (VII) :

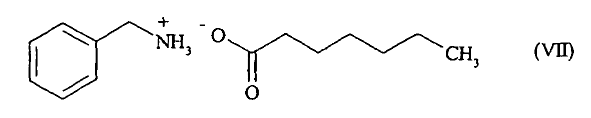

7. Composé de formule (I) qui est le (7-méthoxy-3,4-dihydro-1-naphtalényl)acétonitrile, utile en tant qu'intermédiaire de synthèse de l'agomélatine.

8. Procédé de synthèse de l'agomélatine à partir du composé de formule (I), **caractérisé en ce que** le composé de formule (I) est obtenu par le procédé de synthèse selon l'une quelconque des revendications 1 à 6, et que l'on soumet à une réaction d'aromatisation suivie d'une réaction de réduction puis de couplage avec l'anhydride acétique.

## Claims

1. Process for the industrial synthesis of the compound of formula (I) 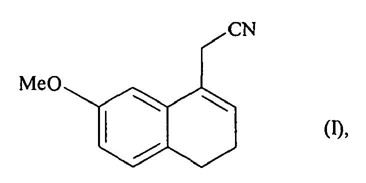 **characterised in that** 7-methoxy-1-tetralone of formula (IV) : 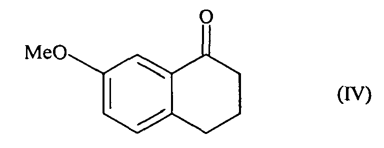 is reacted, under conditions in which the water formed is eliminated, with cyanoacetic acid of formula (V) : 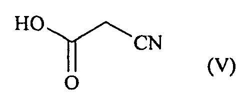 in the presence of a catalytic amount of a compound of formula (VI) : 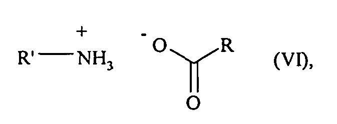 wherein R and R', which may be identical or different, each represents a linear or branched (C₃-C₁₀)alkyl group, an unsubstituted or substituted aryl group or an unsubstituted or substituted, linear or branched aryl-(C₁-C₆)alkyl group,
to yield the compound of formula (I) after filtration and washing with a basic solution, which compound of formula (I) is isolated in the form of a solid after recrystallisation,
wherein:
- aryl is understood to mean a phenyl, naphthyl or biphenyl group,
- the term "substituted" applied to the terms "aryl" and "arylalkyl" signifies that the aromatic moiety of those groups may be substituted by from 1 to 3 identical or different groups selected from linear or branched (C₁-C₆)alkyl, hydroxy and linear or branched (C₁-C₆)alkoxy.

2. Process for the synthesis of the compound of formula (I) according to claim 1, **characterised in that** the reaction is carried out with reflux of toluene.

3. Process for the synthesis of the compound of formula (I) according to claim 1, **characterised in that** the catalyst used is the compound of formula (VIₐ) : 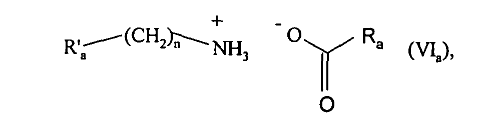 wherein R'ₐ represents a phenyl group unsubstituted or substituted by one or more linear or branched (C₁-C₆)alkyl groups, n is 0 or 1, and Rₐ represents a linear (C₃-C₁₀)-alkyl group.

4. Process for the synthesis of the compound of formula (I) according to claim 1, **characterised in that** R represents a hexyl group.

5. Process for the synthesis of the compound of formula (I) according to claim 1, **characterised in that** R' represents a benzyl group.

6. Process for the synthesis of the compound of formula (I) according to claim 1, **characterised in that** the catalyst used is benzylammonium heptanoate of formula (VII) :

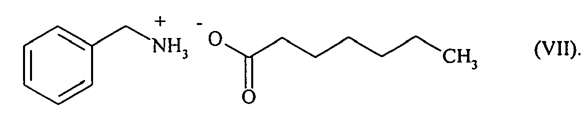

7. Compound of formula (I) which is (7-methoxy-3,4-dihydro-1-naphthalenyl)-acetonitrile, for use as an intermediate in the synthesis of agomelatine.

8. Process for the synthesis of agomelatine starting from the compound of formula (I), **characterised in that** the compound of formula (I) is obtained by the synthesis process according to any one of claims 1 to 6, and is subjected to aromatisation followed by reduction and then to coupling with acetic anhydride.

## Patentansprüche

1. Verfahren zur technischen Synthese der Verbindung der Formel (I) 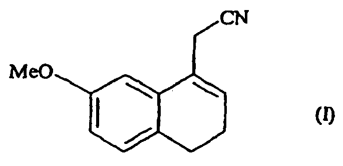 **dadurch gekennzeichnet, daß** man 7-Methoxy-1-tetralon der Formel (IV): 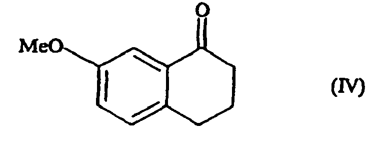 mit Cyanessigsäure der Formel (V): 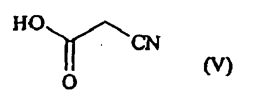 unter Bedingungen zur Abspaltung des gebildeten Wassers in Gegenwart einer katalytischen Menge der Verbindung der Formel (VI): 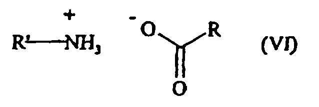 in der R und R', die gleichartig oder verschieden sind, jeweils eine geradkettige oder verzweigte (C₃-C₁₀)-Alkylgruppe, eine nichtsubstituierte oder substituierte Arylgruppe oder eine geradkettige oder verzweigte, nichtsubstituierte oder substituierte Aryl-(C₁-C₆)-alkylgruppe bedeuten,
umsetzt zur Bildung der Verbindung der Formel (I), welche Verbindung der Formel (I) man nach dem Filtrieren und Waschen mit einer basischen Lösung nach der Umkristallisation in Form eines Feststoffs isoliert,
mit der Maßgabe, daß:
- man unter Aryl eine Phenyl-, Naphthyl- oder Biphenylgruppe versteht und
- der Begriff "substituiert" im Hinblick auf die Begriffe «Aryl» und «Arylalkyl» bedeutet, daß der aromatische Teil dieser Gruppen durch 1 bis 3 gleichartige oder verschiedenartige Gruppen ausgewählt aus geradkettigem oder verzweigtem (C₁-C₆)-Alkyl, Hydroxy und geradkettigem oder verzweigtem (C₁-C₆)-Alkoxy substituiert sein kann.

2. Verfahren zur Synthese der Verbindung der Formel (I) nach Anspruch 1, **dadurch gekennzeichnet, daß** man die Umsetzung in Toluol am Rückfluß durchführt.

3. Verfahren zur Synthese der Verbindung der Formel (I) nach Anspruch 1, **dadurch gekennzeichnet, daß** der verwendete Katalysator die Verbindung der Formel (VIₐ) ist: 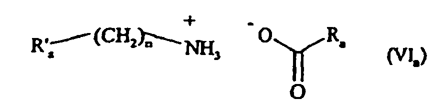 in der R'ₐ eine nichtsubstituierte Phenylgruppe oder eine Phenylgruppe bedeutet, die durch eine oder mehrere geradkettige oder verzweigte (C₁-C₆)-Alkylgruppen substituiert ist, n 0 oder 1 bedeutet und Rₐ eine geradkettige (C₃-C₁₀)-Alkylgruppe darstellt.

4. Verfahren zur Synthese der Verbindung der Formel (I) nach Anspruch 1, **dadurch gekennzeichnet, daß** R eine Hexylgruppe bedeutet.

5. Verfahren zur Synthese der Verbindung der Formel (I) nach Anspruch 1, **dadurch gekennzeichnet, daß** R' eine Benzylgruppe bedeutet.

6. Verfahren zur Synthese der Verbindung der Formel (I) nach Anspruch 1, **dadurch gekennzeichnet, daß** der verwendete Katalysator Benzylammoniumheptanoat der Formel (VII) ist:

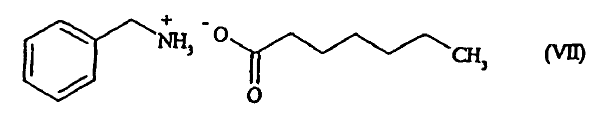

7. Verbindung der Formel (I), nämlich (7-Methoxy-3,4-dihydro-1-naphthalinyl)-acetonitril, das als Zwischenprodukt für die Synthese von Agomelatin nützlich ist.

8. Verfahren zur Synthese von Agomelatin ausgehend von der Verbindung der Formel (I), **dadurch gekennzeichnet, daß** man die Verbindung der Formel (I) nach dem Syntheseverfahren nach einem der Ansprüche 1 bis 6 herstellt und einer A-romatisierungsreaktion, gefolgt von einer Reduktionsreaktion und einer Kupplung mit Essigsäureanhydrid unterwirft.
